# EUROPEAN PATENT SPECIFICATION

(11) **EP 2 228 090 B1**
(45) Date of publication and mention of the grant of the patent: **08.02.2012**
(21) Application number: 09155018.6
(22) Date of filing: 12.03.2009
(51) Int. Cl.: A61M 25/00

(54) **Double lumen catheter**
Doppellumenkatheter
Cathéter à double lumière

(43) Date of publication of application: 15.09.2010
(73) Proprietor: Joline GmbH & Co. KG, 72379 Hechingen (DE)
(72) Inventor: Steegers, Anselm, Dr., 72108 Rottenburg (DE); Schuler, Oliver, 72417 Jungingen (DE)
(74) Representative: Witte, Weller & Partner

(56) References cited:
- WO-A-01/32240
- WO-A-2009/052506
- US-B1- 6 409 700

## Description

The present invention concerns a double lumen catheter for use in the delivery or the withdrawal of fluids to a patient, the catheter comprising an elongated tubular body having a proximal end and distal end, with the distal end being positioned within a vessel of a patient, a divider wall extending along an interior of said tubular body and separating said interior into an intake lumen and a return lumen, the intake lumen and the return lumen each extending from the proximal end to the distal end of the tubular body, with the intake lumen and the return lumen being defined by an outer wall and said divider wall, and said intake lumen and said return lumen each comprising a bevelled opening sloping toward the divider wall and toward the distal end of the tubular body, respectively, the openings being divided by the divider wall, such, that one side of the divider wall faces the intake lumen and the other side of the divider wall faces the return lumen.

Double lumen catheter of this kind are generally known in the state of the art and are used in medical applications, where two separate fluid flow paths are necessary for the treatment of a patient. In particular, the present invention relates to the distal end of a double lumen catheter which contains an opening for fluid intake and/or fluid return.

Various medical applications require the use of a double lumen catheter, with which blood or other fluids can be withdrawn and returned to the body of a patient. They are used, e.g., for hemodialysis, which is a process involving the dialysis of soluble substances and water from the blood by diffusion through semipermeable membranes. Separation of cellular elements and colloids from soluble substances is achieved by different pore size in the membrane and different rates of diffusion. A hemodialyiss device is connected to the body of a patient by means of a catheter. The catheter is - with its distal end placed into the vessel -partially inserted into the body and its proximal end is connected with the hemodialysis machine. During hemodialysis, the blood of a patient flows via the catheter to the extracorporeal circuit consisting of the hemodialysis machine and the blood lines.

In hemodialysis cases where the blood is not adequately treated, a number of adverse effects result from incomplete elimination of toxins from the blood. Therefore, factors such as the degree of recirculation, cardiopulmonary recirculation, access flow and suction pressure developed by the hemodialysis unit as it pulls out blood into the circuit affect the performance of the hemodialysis.

Previously, the conventional way for performing hemodialysis was to use two needles, one for removing the blood from the vessel und for processing the blood in a dialysis machine, and another needle for returning the processed blood into the vessel. When performing this conventional way, care must be taken, that the two needles are spaced apart with a sufficient distance in order to prevent the processed blood from re-entering the blood outlet needle and the blood processing machine. At the same time, the needles must be close enough to one another in order to prevent the vessel from collapsing.

Nowadays, double lumen catheters are state of the art, which have the advantage that the numbers of the tubes that have to be inserted into a patient can be reduced. Further, apertures of intake and outflow/return lumens have been longitudinally spaced apart to prevent cleansed blood from re-entering the blood outlet needle and returning to the dialysis machine. This so-called recirculation reduces the efficiency of the blood treatment process.

High efficiency dialysis requires the use of two lumens with large diameters with an external cross-sectional dimension which is not too large for vascular access. Short-term catheters of the prior art include a simple double-D lumen configuration. The walls are thin, and the equal area lumens make full use of the available space. However, in order to keep its shape during high flow rate dialysis, the catheter is made of a relatively stiff material which is unsuitable for long-term placement. Further, when using stiff material, the risk of causing kinking of the catheter is high if the catheter gets bent more than 180°, resulting in obstruction, splitting and may be responsible for vein penetration injuries.

When using silicone as material for the catheter, which represents a relatively soft and biostable material, thicker walls have to be provided.

Double lumen catheters are generally provided in two principal different tip designs, the one, where only one lumen ends axially at the tip of the catheter, while the other opens to the vessel via side holes. Besides catheters having a double-D lumen configuration, catheters with a double-barrelled circular configuration are known, which are - due to their cross-sectional shape - rather resistant to kinking.

US 5,569,182 discloses a multiple lumen catheter and a method for circulating blood through a multiple lumen catheter, by means of which blood is being withdrawn form a vein trough one of the lumens at a flow rate of at least about 200 mi/min, and inserted into the vein through another of the lumens, the distal ends of which are being spaced apart from one another by no more than about 5 mm.

US 6,409,700 discloses a double lumen catheter, that has an elongated tube with unitary outer walls. A longitudinal planar septum divides the interior of the tube into an inlet lumen and a longer return lumen. The inlet lumen extends from a proximal end of the tube to an end terminating in a distally forward facing aperture. The return lumen extends contiguously with the inlet lumen from the proximal end of the tube to an end terminating in a distally forward facing aperture spaced in the longitudinal direction distally forward of the inlet lumen aperture. A diverting structure extends from an outer wall of the return lumen distally forward of the aperture of the inlet lumen. The diverting structure diverts flow of the treated fluid discharged from the return lumen away from the distally forward facing aperture of the inlet lumen. The blood withdrawn via the catheter is circulated for treatment in a blood purification unit.

US 5,571093 discloses a multiple lumen catheter including a catheter tube having a generally elongate cylindrical body having an axial passageway extending the length thereof. A septum extends across the interior of the body and along the length thereof in order to divide the body into first and second lumens. A bolus extends from a distal end of the body. The bolus has a passage section including an axial and a radial passage portion. The axial passage portion is in fluid communication with the first and second lumens. The radial passage portion includes a single port through the side of the bolus whereby the first and the second lumens are in fluid communication with the port.

WO 01/32240 A1 discloses a bolus tip for a multi-lumen catheter, which tip has a rounded elongate body consisting of an interfacing section and a nose section. The interfacing section includes two separate channels which are separated by a dividing section. The first channel opens to a first bolus U-shaped cavity located between the interfacing section and the nose section. The second channel can terminate at a second bolus cavity directly opposite to the first bolus cavity.

Despite the various solutions suggested and/or provided in the state of the art for double lumen catheters, the problems of recirculation, clotting, inadequate flow rates remain.

Thus, it is an object of the invention to provide an improved double-lumen catheter, which overcomes the problems of the catheters of the state of the art. In particular, the novel catheter is intended to highly prevent recirculation of the blood.

In the catheter mentioned at the outset, this object is achieved by the divider wall at the distal end of the tubular body on both of its two sides comprising at least two ridges extending in the area of the openings and along a longitudinal portion of the tubular body.

The object underlying the invention is achieved in full by this means.

With the at least two ridges extending in the area of the openings and along a longitudinal portion of the tubular body of the catheter, the inflow and/or the return of the blood is directed to the respective opening comprising the ridges. With the at least two ridges at the opening of the intake lumen the intake of the blood flow is actively guided into the lumen. On the other hand - or at the same time - with the at least two ridges being present at the opening of the return lumen, it is assured that the blood leaving the return lumen - and entering the vessel - is not being taken up by the opening of the intake lumen since the ridges provide for a directed outflow of the blood away from the openings of the catheter.

The term "ridge" as used in the present invention includes any elongate rib-, spine-or similar shaped elevation.

The term "catheter" as used in the present invention includes flexible plastic devices as well as rigid devices either made of metal or made of rigid plastics for instance. Preferred materials include polyurethane, polyvinyl chloride, polytetrafluoroethylene, polyethylene, polyamide or the like.

In one embodiment of the invention it is preferred if between two and five ridges are provided, and it is even more preferred if between two and four ridges, that is two, three, or four ridges, are provided.

Thus, the catheter may comprise on the divider wall at the distal end of its tubular body on both of its two sides two, three, four or five ridges extending in the area of the openings and along a longitudinal portion of the tubular body.

The number of the ridges depends on the size of the catheter and in particular on its cross-sectional dimension; thus, a catheter having a rather large cross-section in the area of the openings may comprise more ridges than a catheter having a smaller cross-section in the area of the openings. Further, the number of the ridges may be adapted to the particular use of the catheter, and may be adjusted to provide the desired enhanced and guided inflow/return of the blood.

According to a preferred embodiment of the invention, the at least two ridges are provided in the area of the opening of the intake lumen.

According to another aspect of the invention, the at least two ridges are provided in the area of the opening of the return lumen.

According to yet another aspect of the invention there are provided at least two ridges in the area of the opening of the intake lumen and the return lumen.

In a preferred embodiment, the ridges have different lengths.

This measure has the advantage that the flow characteristics of the blood entering and/or leaving the intake and/or return lumen respectively, can fully be taken into consideration. Thus, in one embodiment of the invention it is preferred with more than one ridge being present in the area of the openings, that one of the ridges extends to the very distal end of the catheter whilst one or more other ridges do not. Similar, according to another embodiment of the invention, and in case the divider wall at the distal end of the tubular body comprises in the area of both, the side facing the intake lumen and the side facing the return lumen, at least two ridges extending in the area of the openings and along a longitudinal portion of the tubular body, one of the ridges may extend from the very distal end of the catheter along a longitudinal portion of the tubular body, whereas one or more others do not, but rather extend from a certain distance of the very end of the catheter along the longitudinal portion of the tubular body.

Further, in a preferred embodiment, the at least two ridges extend generally parallel to the longitudinal axis of the tubular body.

According to another embodiment of the invention, the at least two ridges extend angular with respect to the longitudinal axis of the tubular body.

According to yet another embodiment of the invention, the at least two ridges extend curved with respect to the longitudinal axis of the tubular body. In this embodiment, the ridges can run with a constant or a varying radius. Further, the ridges may extend monotonic or with an inflection point.

With more than one ridge being present, and in case the ridges are extending generally parallel, angular or curved, the at least two ridges may run parallel with respect to one another or not. Further, in case there are provided curved ridges, the curve of the ridges can extend to the same or to the opposite side.

In yet another embodiment, the at least two ridges extend inclined with respect to the divider wall. In this embodiment, the elevation of the ridges is, e.g., in the area of the opening less pronounced than in its extension along the longitudinal axis of the tubular body, i.e. in the direction of lumen, which means that the ridges extend ramp-like. Further, the cross sectional dimensions of the ridges may be such, that they are constant over the entire length of the ridges or that they vary over their length.

In addition, the number, shape and length of ridges in the area of the opening in the intake lumen and the return lumen may be different or the same.

It is to be understood, that the extension of the ridges can be adjusted to the special appliance of the catheter, and may vary depending on the term of the catheterization, the vessel and blood flow characteristics.

According to another preferred embodiment of the invention, the ridges are designed such, that the cross section of one ridge is generally conical shaped, semi-circle shaped, rectangular or wavelike.

Furthermore, in a preferred embodiment, the ridges are designed such, that the cross section of the ridges are identical or different.

According to yet another invention, the intake lumen and/or the return lumen are designed such, that the cross section of the lumen has the form of a semi-circle, a circle, or an irregular shape.

Double and triple lumen catheters are generally provided in two principal different tip designs, the one, where only one lumen ends axially at the tip of the catheter, while the other opens to the vessel via side holes. The other design principle engages two endholes with or without additional side holes, the so called "shot-gun" design.

Double lumen catheter are known having two lumens showing D-shaped cross-sections, those having two circular lumens or for instance one crescent shaped and one circular or oval lumen or lumen cross-sections designed in any irregular shape. In special appliances, it might be preferred if the lumen comprise an irregular shape. In a preferred embodiment, the intake lumen and/or the return lumen are designed, such, that along its extension the cross section of the lumen is uniform or different.

Furthermore, the intake lumen and the return lumen may be designed such, that the cross sections of lumens are the same or different.

The double-lumen catheter according to the invention is used in a method of circulating blood through a double-lumen catheter, the catheter comprising an elongated tubular body having a proximal end and distal end, with the distal end being positioned within a vessel of a patient, a divider wall extending along an interior of said tubular body and separating said interior into an intake lumen and a return lumen, the intake lumen and the return lumen each extending from the proximal end to the distal end of the tubular body, with the intake lumen and the return lumen being defined by an outer wall and said divider wall, and said intake lumen and said return lumen each comprising a bevelled opening sloping toward the divider wall and toward the distal end of the tubular body, respectively, the openings being divided by the divider wall, such, that one side of the divider wall faces the intake lumen and the other side of the divider wall faces the return lumen, wherein the divider wall at the distal end of the tubular body on at both of its two sides comprises at least two ridges extending in the area of the openings and along a longitudinal portion of the tubular body. The method comprises the steps of withdrawing blood from the vessel through one of the lumens and inserting blood into said vessel through the other lumen.

Thus, the invention also concerns the catheter according to the invention for use in the withdrawing and delivery of fluids to a patient. The catheter according to the invention is particularly suited for use in hemodialysis.

It is to be understood, that the term "vessel" includes blood vessels, in particular veins.

Further advantages and features will become evident from the following description and from the attached drawings.

It will be appreciated that the aforementioned features and those still to be explained below can be used not only in the respectively cited combination but also in other combinations or on their own, without departing from the scope of the present invention.

### Brief description of the drawings

- Fig. 1: shows the tip of a catheter according to one embodiment of the inven- tion, with a) representing a plan view, showing the opening of the in- take lumen; b) representing a side view; and c) representing a plan view of the opening of the return lumen.
- Fig. 2: shows view of the longitudinal section of the tip of Fig. 1 (a); and a cross sectional view of the catheter according to an embodiment of the invention along the line x -> x' (b);
- Fig. 3: shows schematic plan views of different embodiments of the catheter according to the invention: a) an embodiment with two ridges having different length; b) an embodiment with two ridges running slightly curved, with the curve in opposite directions; c) an embodiment with two ridges running parallel; d) an embodiment with two ridges not running parallel; and
- Fig. 4:: shows cross-sectional views of the ridges of embodiments according to the invention: a) the ridges being irregularly shaped; b) the ridges hav- ing a triangular cross-section; c) the ridges having a rectangular cross- section; d) the ridges having a semicircular cross section.

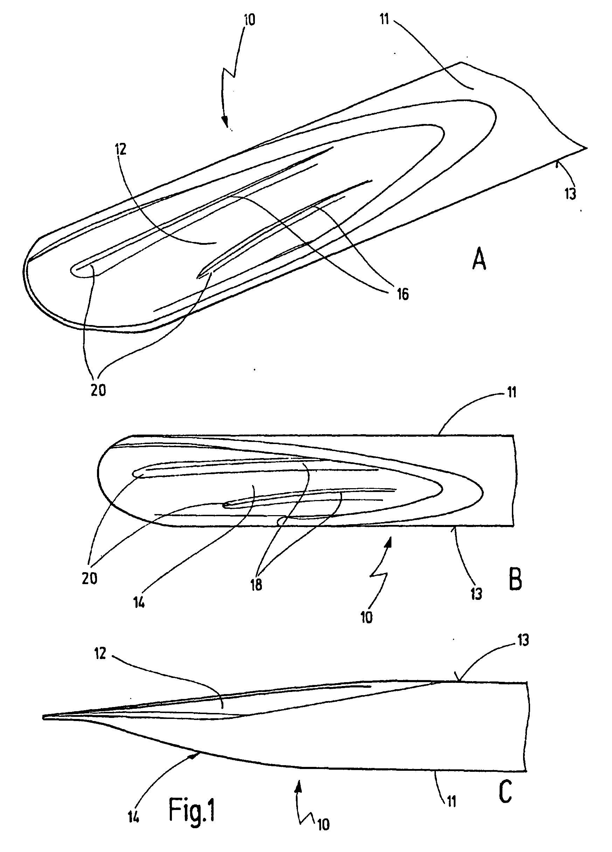

Fig. 1 shows the distal end of a double lumen catheter according to an embodiment of the invention, with its elongated tubular body 11 being depicted in part. In Fig. 1, a double lumen catheter tip 10 of an embodiment according to the invention is shown, with the opening of intake lumen 22 being designated as 12, as it is depicted in Fig. 1a, and the opening of return lumen 24 as 14, as it is depicted in Fig. 1b. The openings 12 and 14 are separated via a divider wall (not pictured in Fig. 1). Fig. 1c shows a side view of the tip 10, showing the openings 12 and 14 of both, the intake 22 and the return lumen 24. In the embodiment shown in Fig. 1, the tip 10 comprises ridges 16 and 18, extending in the area of the openings 12 and 14 of the intake 22 and the return lumen 24 and along a longitudinal portion of the tubular body. In the embodiment shown in Fig. 1, the distal ends 20 of the ridges 16 start to raise/incline in a certain, short distance from the very end of the tip. As can be seen in Fig. 1c, the openings 12 an 14 of the lumens 22, 24 represent beveled edges of the lumens 22, 24, sloping outwardly and away from the catheter tip, which can be inserted percutaneously.

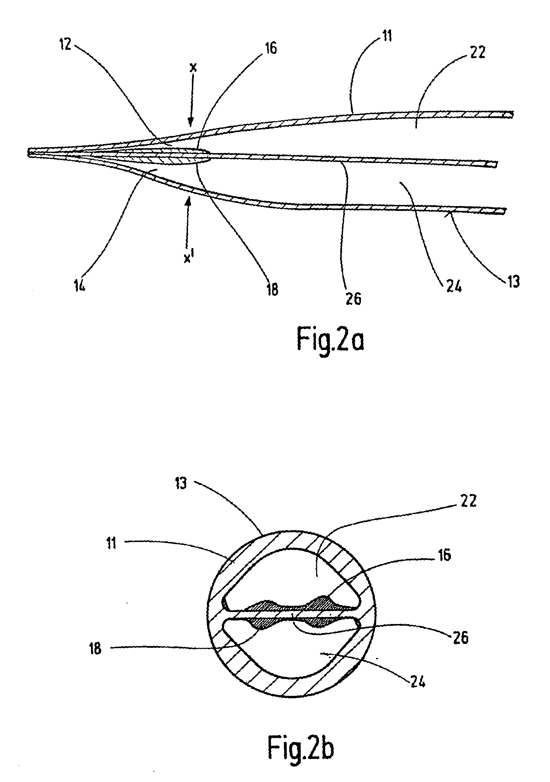

Fig. 2a shows a view of longitudinal section of the catheter tip of Fig. 1, with ends of the lumens 22 and 24 having beveled edges, which represent the openings 12 , 14 of the lumens 22 and 24, respectively. The lumens 22 and 24 are separated by divider wall 26, extending along the interior of the catheter and separating said interior into intake lumen 22 and return lumen 24. The intake and the return lumen 22, 24, are, thus, defined by the divider all 26 and outer wall 13 of the elongated tubular body 11. Also, in Fig. 2a the ridges 16 and 18, being located at the divider wall 26, are shown, extending in the opening areas of the lumen 22 and 24.

Fig. 2b shows a cross sectional view along the line x -> x' of Fig. 2a, depicting that the shape of the ridges 16, 18, is - in cross section - about half circular.

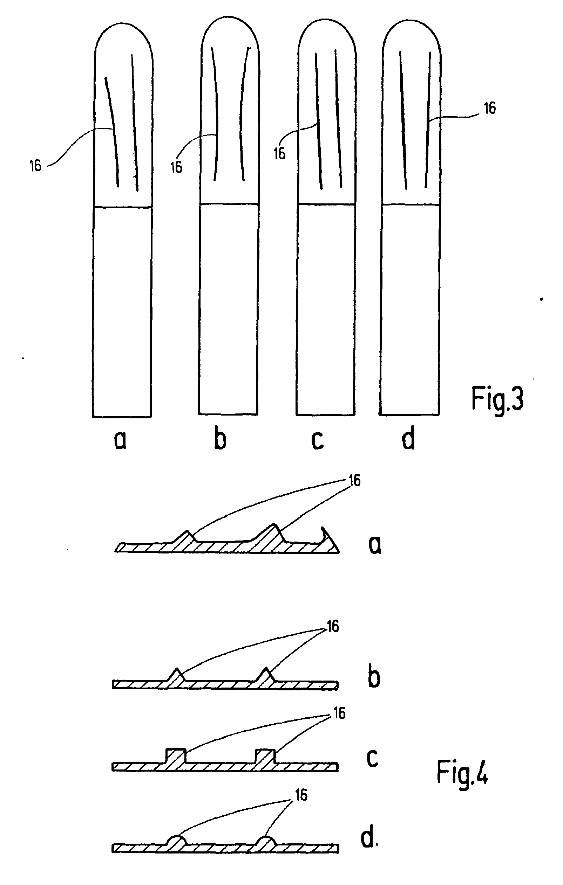

Figs. 3 and 4 show schematic drawings of embodiments of the catheter tip according to the invention, according to which the ridges 16 and/or 18 can be of different length (Fig. 3a), can run slightly curved in respect to the longitudinal axis of the catheter, with the curve in opposite directions (Fig. 3b), can run in parallel (Fig. 3c), or nonparallel.

Further, and this is shown in the schematic drawings of Figs. 4, the catheter tip according to the invention may have ridges, which are irregularly shaped, as depicted in Fig. 4a), which have a triangular cross-section (Fig. 4b), which have a rectangular cross-section (Fig. 4c), and which have a semicircular cross section (Fig. 4d). Of course, the ridges may comprise any kind of other shape, which serves the purpose of supporting to direct the flow into the intake lumen and/or out of the return lumen.

The operating of the catheter will be described with reference to a double lumen catheter according to the present invention. The distal end of the double lumen catheter, i.e. its tip 10, is inserted into, e.g., the vein of a patient to be treated at a slight angle in the direction of blood flow. The catheter may have to be moved slightly inwardly or outwardly in order to achieve optimum blood flow because of the beveled edges. The proximal end of the catheter is connected to, e.g., a dialysis machine. With the machine in operation, blood flows from the vein into the intake lumen 22 via the outwardly sloping beveled edge, i.e. the opening 12, to the dialysis machine, where the blood is being processed. Subsequently, the processed blood is returned to the patient through the return lumen 24 via its outwardly sloping beveled edge, i.e. opening 14, at a pressure to overcome natural resistance to the returning blood. By means of the ridges 16, the blood flow from the vein is systematically directed and guided into the intake lumen 22, and, on the other hand, by means of the ridges 18, out of the return lumen 24, again. The ridges 16, 18 also provide for the blood flow that re-enters the vein via the return lumen 24 does not again enter the intake lumen 22, but rather guide the processed blood away from the opening 12 of the intake lumen 22 in order to guarantee the processed blood being delivered to areas downstream of the insertion of the tip10.

## Claims

1. A double-lumen catheter for use in the delivery or the withdrawal of fluids to a patient, the catheter comprising
- an elongated tubular body (11) having a proximal end and distal end, with the distal end positionable within a vessel of a patient,
- a divider wall (26) extending along an interior of said tubular body (11) and separating said interior into an intake lumen (22) and a return lumen (24),
- the intake lumen (22) and the return lumen (24) each extending from the proximal end to the distal end of the tubular body (11), with the intake lumen (22) and the return lumen (24) being defined by an outer wall (13) and said divider wall (26), and
- said intake lumen (22) and said return lumen (24) each comprising a bevelled opening (12; 14) sloping toward the divider wall and toward the distal end of the tubular body, respectively, the openings (12; 14) being divided by the divider wall (26), such, that one side of the divider wall (26) faces the intake lumen (22) and the other side of the divider wall (26) faces the return lumen (24),
**characterized in that** the divider wall (26) at the distal end of the tubular body (11) on both of its two sides comprises at least two ridges (16; 18) extending in the area of the openings (12; 14) and along a longitudinal portion of the tubular body (11).

2. The double-lumen catheter as claimed in claim 1, **characterized in that** between two and five ridges (16; 18) are provided.

3. The double-lumen catheter as claimed in claim 1 or 2, **characterized in that** between two and four ridges (16; 18) are provided.

4. The double-lumen catheter as claimed in claim 3, **characterized in that** the ridges (16; 18) have different lengths.

5. The double-lumen catheter as claimed in any of claims 1 to 4, **characterized in that** the at least two ridge (16; 18) extends generally parallel to the longitudinal axis of the tubular body (11).

6. The double-lumen catheter as claimed in any of claims 1 to 4, **characterized in that** the at least two ridge (16; 18) extends angular with respect to the longitudinal axis of the tubular body (11).

7. The double-lumen catheter as claimed in any of claims 1 to 4, **characterized in that** the at least two ridge (16; 18) extends curved with respect to the longitudinal axis of the tubular body (11).

8. The double-lumen catheter as claimed in any of claims 1 to 6, **characterized in that** the at least two ridge (16; 18) is inclined with respect to the divider wall (26).

9. The double-lumen catheter as claimed in any of claims 1 to 8, **characterized in that** the at least two ridge (16; 18) is designed such, that the cross section of the at least two ridge (16; 18) is generally conical shaped, semi-circle shaped, rectangular or irregular.

10. The double-lumen catheter as claimed in any of claims 3 to 9, **characterized in that** the ridges (16; 18) are designed such, that the cross section of the ridges (16; 18) are identical or different.

11. The double-lumen catheter as claimed in any of claims 1 to 9, **characterized in that** the intake lumen (22) and/or the return lumen (24) are designed such, that the cross section of the lumen (22; 24) has the form of a semi-circle or a circle.

12. The double-lumen catheter as claimed in any of claims 1 to 11, **characterized in that** the intake lumen (22) and/or the return lumen (24) are designed, such, that along its extension the cross section of the lumen (22; 24) is uniform or different.

13. The double-lumen catheter as claimed in any of claims 1 to 12, **characterized in that** the intake lumen (22) and the return lumen (24) are designed such, that the cross sections of lumens (22; 24) are the same or different.

14. The double-lumen catheter as claimed in any of claims 1 to 13, **characterized in that** the divider wall (26) at the distal end of the tubular body (11) on the side facing the intake lumen (22) comprises at least two ridge (16) extending in the area of the openings (12) and along a longitudinal portion of the tubular body (11).

15. The double-lumen catheter as claimed in any of claims 1 to 13, **characterized in that** the divider wall (26) at the distal end of the tubular body (11) on the side facing the return lumen (24) comprises at least two ridge (18) extending in the area of the openings (14) and along a longitudinal portion of the tubular body (11).

16. The double-lumen catheter as claimed in any of claims 1 to 15, **characterized in that** the divider wall (26) at the distal end of the tubular body (11) comprises on both, the side facing the intake lumen (22) and the side facing the return lumen (24), at least two ridge (16; 18) extending in the area of the openings (12; 14) and along a longitudinal portion of the tubular body (11).

## Patentansprüche

1. Doppellumenkatheter zur Verwendung in der Abgabe oder der Entnahme von Fluiden an einen Patienten, wobei der Katheter Folgendes aufweist:
- einen länglichen tubulären Körper (11) mit einem proximalen Ende und einem distalen Ende, wobei das distale Ende in einem Gefäß eines Patienten positionierbar ist,
- einer Trennwand (26), die sich entlang des Inneren des tubulären Körpers (11) erstreckt, und die das Innere in ein Aufnahmelumen (22) und ein Rückführlumen (24) trennt,
- das Aufnahmelumen (22) und das Rückführlumen (24) erstrecken sich jeweils vom proximalen Ende zum distalen Ende des tubulären Körpers (11), wobei das Aufnahmelumen (22) und das Rückführlumen (24) durch eine äußere Wand (13) und die Trennwand (26) definiert werden, und
- das Aufnahmelumen (22) und das Rückführlumen (24) umfassen jeweils eine angeschrägte Öffnung (12; 14), die jeweils in Richtung der Trennwand und in Richtung des distalen Endes des tubulären Körpers abschrägt, wobei die Öffnungen (12; 14) durch die Trennwand (26) derart getrennt sind, dass eine Seite der Trennwand (26) dem Aufnahmelumen (22) gegenüberliegt und dass die andere Seite der Trennwand (26) dem Rückführlumen (24) gegenüberliegt,
**dadurch gekennzeichnet, dass** die Trennwand (26) am distalen Ende des tubulären Körpers (11) auf beiden seiner zwei Seiten zumindest zwei Rippen (16; 18) aufweist, die sich im Bereich der Öffnungen (12; 14) und entlang eines länglichen Abschnitts des tubulären Körpers (11) erstrecken.

2. Doppellumenkatheter nach Anspruch 1, **dadurch gekennzeichnet, dass** zwischen zwei und fünf Rippen (16; 18) vorliegen.

3. Doppellumenkatheter nach Anspruch 1 oder 2, **dadurch gekennzeichnet, dass** zwischen zwei und vier Rippen (16; 18) vorgesehen sind.

4. Doppellumenkatheter nach Anspruch 3, **dadurch gekennzeichnet, dass** die Rippen (16; 18) unterschiedliche Längen besitzen.

5. Doppellumenkatheter nach einem der Ansprüche 1 bis 4, **dadurch gekennzeichnet, dass** sich die zumindest zwei Rippen (16; 18) im Wesentlichen parallel zur länglichen Achse des tubulären Körpers (11) erstrecken.

6. Doppellumenkatheter nach einem der Ansprüche 1 bis 4, **dadurch gekennzeichnet, dass** sich die zumindest zwei Rippen (16; 18) angewinkelt bezüglich der länglichen Achse des tubulären Körpers (11) erstrecken.

7. Doppellumenkatheter nach einem der Ansprüche 1 bis 4, **dadurch gekennzeichnet, dass** sich die zumindest zwei Rippen (16; 18) bezüglich der länglichen Achse des tubulären Körpers (11) kurvenförmig erstrecken.

8. Doppellumenkatheter nach einem der Ansprüche 1 bis 6, **dadurch gekennzeichnet, dass** die zumindest zwei Rippen (16; 18) bezüglich der Trennwand (26) schräg sind.

9. Doppellumenkatheter nach einem der Ansprüche 1 bis 8, **dadurch gekennzeichnet, dass** die zumindest zwei Rippen (16; 18) derart ausgebildet sind, dass der Querschnitt der zumindest zwei Rippen (16; 18) im Wesentlichen konisch, halbkreisförmig, rechteckig oder unregelmäßig ist.

10. Doppellumenkatheter nach einem der Ansprüche 3 bis 9, **dadurch gekennzeichnet, dass** die Rippen (16; 18) derart ausgebildet sind, dass der Querschnitt der Rippen (16; 18) identisch oder unterschiedlich ist.

11. Doppellumenkatheter nach einem der Ansprüche 1 bis 9, **dadurch gekennzeichnet, dass** das Aufnahmelumen (22) und/oder das Rückführlumen (24) derart ausgebildet ist, dass der Querschnitt der Lumen (22; 24) die Form eines Halbkreises oder eines Kreises hat.

12. Doppellumenkatheter nach einem der Ansprüche 1 bis 11, **dadurch gekennzeichnet, dass** das Aufnahmelumen (22) und/oder das Rückführlumen (24) derart ausgebildet ist, dass der Querschnitt über den Verlauf des Lumens (22; 24) einheitlich oder unterschiedlich ist.

13. Doppellumenkatheter nach einem der Ansprüche 1 bis 12, **dadurch gekennzeichnet, dass** das Aufnahmelumen (22) und das Rückführlumen (24) derart ausgebildet sind, dass die Querschnitte der Lumen (22; 24) gleich oder unterschiedlich sind.

14. Doppellumenkatheter nach einem der Ansprüche 1 bis 13, **dadurch gekennzeichnet, dass** die Trennwand (26) am distalen Ende des tubulären Körpers (11) auf der Seite, die dem Aufnahmelumen (22) gegenüberliegt, zumindest zwei Rippen (16) aufweist, die sich im Bereich Öffnungen (12) und entlang einem länglichen Abschnitt des tubulären Körpers (11) erstrecken.

15. Doppellumenkatheter nach einem der Ansprüche 1 bis 13, **dadurch gekennzeichnet, dass** die Trennwand (26) am distalen Ende des tubulären Körpers (11) auf der Seite, die dem Rückführlumen (24) gegenüberliegt, zumindest zwei Rippen (18) aufweist, die sich im Bereich der Öffnungen (14) und entlang eines länglichen Abschnitts des tubulären Körpers (11) erstrecken.

16. Doppellumenkatheter nach einem der Ansprüche 1 bis 15, **dadurch** gekennzeichnen, die Trennwand (26) arm distalen des tubulären Körpers (11) auf beiden Seiten, nämlich der Seite, die dem Aufnahmelumen (22) gegenüberliegt, und auf der Seite, die dem Rückführlumen (24) gegenüberliegt, zumindest Rippen (16; 18) aufweist, die sich im Bereich der Öffnungen (12; 14) und entlang eines länglichen Abschnitts des tubulären Körpers (11) erstrecken.

## Revendications

1. Cathéter à deux lumières destiné à être utilisé pour l'administration ou le prélèvement de fluides chez un patient, le cathéter comprenant
- un corps tubulaire allongé (11) ayant une extrémité proximale et une extrémité distale, l'extrémité distale pouvant être positionnée à l'intérieur d'un vaisseau d'un patient,
- une paroi de division (26) s'étendant le long d'un intérieur dudit corps tubulaire (11) et divisant ledit intérieur en une lumière d'admission (22) et une lumière de retour (24),
- la lumière d'admission (22) et la lumière de retour (24) s'étendant chacune de l'extrémité proximale à l'extrémité distale du corps tubulaire (11), la lumière d'admission (22) et la lumière de retour (24) étant définies par une paroi extérieure (13) et ladite paroi de division (26), et
- ladite lumière d'admission (22) et ladite lumière de retour (24) comprenant chacune une ouverture en biseau (12 ; 14) descendant en pente vers la paroi de division et vers l'extrémité distale du corps tubulaire, respectivement, les ouvertures (12 ; 14) étant divisées par la paroi de division (26), de telle sorte qu'un côté de la paroi de division (26) est tourné vers la lumière d'admission (22) et l'autre côté de la paroi de division (26) est tourné vers la lumière de retour (24),
**caractérisé en ce que** la paroi de division (26) au niveau de l'extrémité distale du corps tubulaire (11) comprend sur chacun de ses deux côtés au moins deux nervures (16 ; 18) s'étendant dans la zone des ouvertures (12 ; 14) et le long d'une partie longitudinale du corps tubulaire (11).

2. Cathéter à deux lumières selon la revendication 1, **caractérisé en ce que** deux à cinq nervures (16 ; 18) sont fournies.

3. Cathéter à deux lumières selon la revendication 1 ou 2, **caractérisé en ce que** deux à quatre nervures (16 ; 18) sont fournies.

4. Cathéter à deux lumières selon la revendication 3, **caractérisé en ce que** les nervures (16 ; 18) ont différentes longueurs.

5. Cathéter à deux lumières selon l'une quelconque des revendications 1 à 4, **caractérisé en ce que** les deux nervures ou plus (16 ; 18) s'étendent généralement parallèlement à l'axe longitudinal du corps tubulaire (11).

6. Cathéter à deux lumières selon l'une quelconque des revendications 1 à 4, **caractérisé en ce que** les deux nervures ou plus (16 ; 18) s'étendent en formant un angle par rapport à l'axe longitudinal du corps tubulaire (11).

7. Cathéter à deux lumières selon l'une quelconque des revendications 1 à 4, **caractérisé en ce que** les deux nervures ou plus (16 ; 18) s'étendent en formant une courbe par rapport à l'axe longitudinal du corps tubulaire (11).

8. Cathéter à deux lumières selon l'une quelconque des revendications 1 à 6, **caractérisé en ce que** les deux nervures ou plus (16 ; 18) sont inclinées relativement à la paroi de division (26).

9. Cathéter à deux lumières selon l'une quelconque des revendications 1 à 8, **caractérisé en ce que** les deux nervures ou plus (16 ; 18) sont conçues de manière à ce que la section transversale des deux nervures ou plus (16 ; 18) soit généralement de forme conique, semi-circulaire, rectangulaire ou irrégulière.

10. Cathéter à deux lumières selon l'une quelconque des revendications 3 à 9, **caractérisé en ce que** les nervures (16 ; 18) sont conçues de manière à ce que la section transversale des nervures (16 ; 18) soit identique ou différente.

11. Cathéter à deux lumières selon l'une quelconque des revendications 1 à 9, **caractérisé en ce que** la lumière d'admission (22) et/ou la lumière de retour (24) sont conçues de manière à ce que la section transversale des lumières (22 ; 24) ait une forme semi-circulaire ou circulaire.

12. Cathéter à deux lumières selon l'une quelconque des revendications 1 à 11, **caractérisé en ce que** la lumière d'admission (22) et/ou la lumière de retour (24) sont conçues de manière à ce que, le long de son extension, la section transversale des lumières (22 ; 24) soit uniforme ou différente.

13. Cathéter à deux lumières selon l'une quelconque des revendications 1 à 12, **caractérisé en ce que** la lumière d'admission (22) et la lumière de retour (24) sont conçues de manière à ce que les sections transversales des lumières (22 ; 24) soient identiques ou différentes.

14. Cathéter à deux lumières selon l'une quelconque des revendications 1 à 13, **caractérisé en ce que** la paroi de division (26) au niveau de l'extrémité distale du corps tubulaire (11) sur le côté faisant face à la lumière d'admission (22) comprend au moins deux nervures (16) s'étendant dans la zone des ouvertures (12) et le long d'une partie longitudinale du corps tubulaire (11).

15. Cathéter à deux lumières selon l'une quelconque des revendications 1 à 13, **caractérisé en ce que** la paroi de division (26) au niveau de l'extrémité distale du corps tubulaire (11) sur le côté faisant face à la lumière de retour (24) comprend au moins deux nervures (18) s'étendant dans la zone des ouvertures (14) et le long d'une partie longitudinale du corps tubulaire (11).

16. Cathéter à deux lumières selon l'une quelconque des revendications 1 à 15, **caractérisé en ce que** la paroi de division (26) au niveau de l'extrémité distale du corps tubulaire (11) comprend, à la fois sur le côté faisant face à la lumière d'admission (22) et sur le côté faisant face à la lumière de retour (24), au moins deux nervures (16 ; 18) s'étendant dans la zone des ouvertures (12 ; 14) et le long d'une partie longitudinale du corps tubulaire (11).
